# EUROPEAN PATENT SPECIFICATION

(11) **EP 1 503 746 B1**
(45) Date of publication and mention of the grant of the patent: **10.10.2007**
(21) Application number: 03731699.9
(22) Date of filing: 22.01.2003
(51) Int. Cl.: A61K 31/196, A61P 29/00

(54) **PHARMACEUTICAL USE OF COX-2 INHIBITORS IN ANGIOGENESIS-MEDIATED OCULAR DISORDERS**
PHARMAZEUTISCHE VERWENDUNG VON COX-2-HEMMERN IN ANGIOGENESE-VERMITTELTEN AUGENERKRANKUNGEN
UTILISATION PHARMACEUTIQUE D'INHIBITEURS DE COX-2 DANS DES AFFECTIONS OCULAIRES INDUITES PAR L'ANGIOGENESE

(30) Priority: 23.01.2002 GB 0201520
(43) Date of publication of application: 09.02.2005
(73) Proprietor: Novartis AG, 4056 Basel (CH); Novartis Pharma GmbH, 1235 Vienna (AT)
(72) Inventor: BRAZZELL, Romulus, Kimbro, Alpharetta, GA 30201 (US); LAMBROU, George, N., F-68870 Bartenheim-La Chaussée (FR); LATOUR, Elisabeth, F-68870 Bartenheim-La Chaussée (FR); OTTLECZ, Anna, CH-4055 Basel (CH); WOOD, Jeanette, Marjorie, CH-4105 Biel-Benken (CH)
(74) Representative: de Weerd, Petrus G.W.
(86) International application number: PCT/EP2003/000612
(87) International publication number: WO 2003/061645

(56) References cited:
- WO-A-02/20090
- WO-A-99/11605
- WO-A-03/020261
- US-A- 3 652 762
- MOSER P ET AL: "SYNTHESIS AND QUANTITATIVE STRUCTURE-ACTIVITY RELATIONSHIPS OF DICLOFENAC ANALOGUES" JOURNAL OF MEDICINAL CHEMISTRY, AMERICAN CHEMICAL SOCIETY. WASHINGTON, US, vol. 33, 1990, pages 2358-2368, XP001024801 ISSN: 0022-2623

## Description

This invention relates to selective cyclooxygenase-2 inhibitors (COX-2 Inhibitors), in particular to the use of COX-2 inhibitors in the treatment of angiogenesis-related ocular diseases wherein said anglogenesis-mediated disease is selected from age-related macular degeneration, diabetic retinopathy and diabetic macular edema.

COX-2 inhibitors and their use as non-steroidal anti-inflammatory drugs (NSAIDs) for the treatment of inflammatory diseases and pain are well known in the art. Further it has been proposed (see for instance USP 6,025,353, Searle) to use COX-2 inhibitors to treat angiogenesis related disorders including ophthalmic conditions such as corneal graft rejection, ocular neovascularisation, retinal neovascularisation, including neovascularisation following injury or infection, diabetic retinopathy, retrolental fibroplasias, and neovascular glaucoma.

It has now been found that certain COX-2 inhibitors, in particular 5-alkyl substituted 2-arylaminophenylacetic acid derivative COX-2 inhibitors, have desirable properties for use in the treatment of ocular neovascular disease.

Accordingly the invention provides a method of treating an angiogenesis-mediated ocular disorder in a subject in need of such treatment which comprises administering to the subject an effective amount of a COX-2 inhibitor of formula I 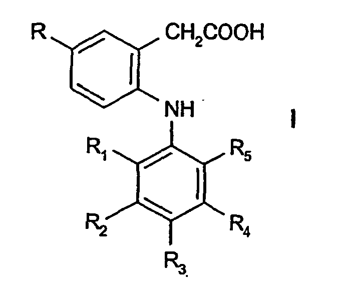 wherein
R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl;
a pharmaceutically acceptable salt thereof; or
a pharmaceutically acceptable prodrug ester thereof.

Further the invention provides the use of a compound of formula I (or pharmaceutically acceptable salt or prodrug ester thereof) as defined above for the preparation of a medicament, for use in the treatment of an angiogenesis-mediated ocular disorder, wherein said anglogenesis-mediated disease is selected from age-related macular degeneration, diabetic retinopathy and diabetic macular edema.

Angiogenesis-mediated ocular disorders which may be treated according to the invention include ocular diseases and disorders which directly or indirectly involve angiogenesis or neovascularisation, such as age-related macular degeneration, diabetic retinopathy.

In the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

Particularly preferred compounds of formula I are those wherein R is methyl or ethyl; R₁ is chloro or fluoro; R₂ is hydrogen; R₃ is hydrogen, fluoro, chloro, methyl or hydroxy; R₄ is hydrogen; and R₅ is chloro, fluoro or methyl; pharmaceutically acceptable salts thereof; and pharmaceutically acceptable esters thereof.

A particularly preferred embodiment relates to the compounds of formula I wherein R is methyl or ethyl; R₁ is fluoro; R₂ is hydrogen; R₃ is hydrogen, fluoro or hydroxy; R₄ is hydrogen; and R₅ is chloro; pharmaceutically acceptable salts thereof; and pharmaceutically acceptable prodrug esters thereof.

Another particularly preferred embodiment of the invention relates to compounds of formula I wherein R is ethyl or methyl; R₁ is fluoro; R₂ is hydrogen or fluoro; R₃ is hydrogen, fluoro, ethoxy or hydroxy; R₄ is hydrogen or fluoro; and R₅ is chloro, fluoro or methyl; pharmaceutically acceptable salts thereof; and pharmaceutically acceptable prodrug esters thereof.

Further are said compounds wherein R is methyl or ethyl; R₁ is fluoro; R₂-R₄ are hydrogen or fluoro; and R₅ is chloro or fluoro; pharmaceutically acceptable salts thereof; and pharmaceutically acceptable prodrug esters thereof.

A further embodiment of the invention relates to the compounds of formula I wherein R is methyl or ethyl; R₁ is fluoro; R₂ is fluoro; R₃ is hydrogen, ethoxy or hydroxy; R₄ is fluoro; and R₅ is fluoro; pharmaceutically acceptable salts thereof; and pharmaceutically acceptable prodrug esters thereof.

Another embodiment of the invention relates to the compounds of formula I wherein R is methyl; R₁ is fluoro; R₂ is hydrogen; R₃ is hydrogen or fluoro; R₄ is hydrogen; and R₅ is chloro; pharmaceutically acceptable salts thereof; and pharmaceutically acceptable prodrug esters thereof.

Particularly preferred embodiments of the invention relate to compounds of formula I
(a) wherein R is methyl; R₁ is fluoro; R₂ is hydrogen; R₃ is hydrogen; R₄ is hydrogen; and R₅ is chloro; pharmaceutically acceptable salts thereof; and pharmaceutically acceptable prodrug esters thereof;
(b) wherein R is methyl; R₁ is fluoro; R₂ is hydrogen; R₃ is fluoro; R₄ is hydrogen; and R₅ is chloro; pharmaceutically acceptable salts thereof; and pharmaceutically acceptable prodrug esters thereof;
(c) wherein R is ethyl; R₁ is fluoro; R₂ is fluoro; R₃ is hydrogen; R₄ is fluoro; and R₅ is fluoro; pharmaceutically acceptable salts thereof; and pharmaceutically acceptable prodrug esters thereof; and
(d) wherein R is ethyl; R₁ is chloro; R₂ is hydrogen; R₃ is chloro; R₄ is hydrogen; and R₅ is methyl; pharmaceutically acceptable salts thereof; and pharmaceutically acceptable prodrug esters thereof.

Most preferably 5-methyl-2-(2'-chloro-6'-fluoroanilino)-phenylacetic acid or a pharmaceutically acceptable salt thereof, or a pharmaceutically acceptable prodrug thereof is used as the COX-2 inhibitor of the invention.

Pharmacologically acceptable salts of the compounds of formula I are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Pharmaceutically acceptable prodrug esters of the compounds of formula I are ester derivatives which are convertible by solvolysis or under physiological conditions to the free carboxylic acids of formula I. Such esters are e.g. lower alkyl esters (such as the methyl or ethyl ester), carboxy-lower alkyl esters such as the carboxymethyl ester, nitrooxy-lower alkyl esters (such as the 4-nitrooxybutyl ester), and the like. Preferred prodrugs are the compounds of formula Ia 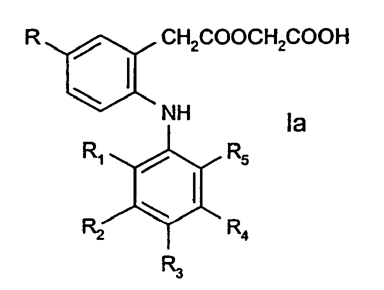 wherein R and R₁-R₅ have meaning as defined hereinabove for compounds of formula I; and pharmaceutically acceptable salts thereof.

Compounds of formula I and Ia and their synthesis are described in published international patent applications Nos. WO 99/11605 and WO 01/23346.

The COX-2 inhibitor compounds of formula I and pharmaceutically acceptable salts and esters thereof, are preferably used in the form of pharmaceutical compositions comprising an effective amount thereof in conjunction or admixture with excipients or carriers suitable for either enteral, parenteral or topical application. In addition, they may also contain other therapeutically valuable substances. The compositions may be prepared according to conventional mixing, granulating or coating methods, respectively, and contain about 0.1 to 90%, preferably about 1 to 60%, of the active ingredient.

Pharmaceutical compositions comprising the compounds of formula I may be, for example, compositions for enteral, such as oral, rectal, aerosol inhalation or nasal administration; compositions for parenteral, such as intravenous or subcutaneous administration; compositions for transdermal administration (e.g. passive or iontophoretic), or compositions for topical administration,

Preferably, the pharmaceutical compositions comprising the compounds of formula I are adapted to oral or topical administration.

The particular mode of administration and the dosage may be selected by the attending physician taking into account the particulars of the patient, especially age, weight, life style, activity level, etc.

Preferred oral forms are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salt and/or polyethyleneglycol; for tablets also c) binders e.g. magnesium aluminum silicate, starch paste, gelatin, tragacanth, methylcellulose, sodium carboxymethylcellulose and or polyvinylpyrrolidone; if desired d) disintegrants, e.g. starches, agar, alginic acid or its sodium salt, or effervescent mixtures; and/or e) absorbents, colorants, flavors and sweeteners. Tablets may be either film coated or enteric coated according to methods known in the art.

Suitable injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers.

Suitable formulations for transdermal application include an effective amount of a compound of the invention with carrier. Advantageous carriers include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Preferred formulations for topical application, e.g. to the skin and eyes, include aqueous solutions, suspensions, ointments, creams, gels or sprayable formulations, for example, for delivery by aerosol or the like. Such topical formulations typically contain from about 0.1 up to about 50% by weight, preferably from about 1 up to about 20% by weight of COX-2 inhibitor compound.

The dosage of COX-2 inhibitor administered is dependent on the subject, the body weight, age and individual condition, and on the form of administration. A unit dosage for oral administration to a mammal of about 50 to 70 kg weight may contain between about 5 and 2000 mg, e.g. from 100-800 mg, preferably 200-400 mg of the active ingredient. When the COX-2 inhibitor is 5-methyl-2-(2'-chloro-6'-fluoro-anilino)-phenyl acetic acid, or a pharmaceutically acceptable salt thereof, an appropriate dose is in the range from 100 to 1500 mg of 5-methyl-2-(2'-chloro-6'-fluoro-anilino)-phenyl acetic acid daily, for example, 200-1000 mg/day, such as 200, 400, 500, 600, 800, 900 or 1000 mg/day, administered in one or two doses daily.

Parenteral formulations are especially injectable fluids that are effective in various manners, such as intravenously, intramuscularly, intraperitoneally, intranasally, intradermally or subcutaneously.

Pharmaceutical compositions comprising compounds of formula I for use in accordance with invention are conveniently supplied in the form of a package comprising the composition, e.g. as a single dose or multiple doses thereof or as a reservoir of composition for repated application (e.g. for topical application), together with written instructions or other indications (e.g. a package insert) for use in the treatment of an angiogenesis-mediated ocular disorder.

The Compounds of formula I may be used with other antiangiogenic agents including bisphophonates, e.g. ibandronate, alendronate or zoledronate and salts and esters thereof, EGFR antagonists.

Preferred embodiments of the instant invention include uses, as described above, wherein the compound of formula (I), in particular 5-methyl-2-(2'-chloro-6'-fluoroanilino)-phenylacetic acid, pharmaceutically acceptable salts thereof; or pharmaceutically acceptable prodrug esters thereof, is in the form of an oral composition and the angiogenesis-mediated ocular disorder is selected from age-related macular degeneration, diabetic retinopathy and diabetic macular edema, more preferably from age-related macular degeneration and diabetic retinopathy.

The ocular antiangiogenic properties of compounds of formula I may be demonstrated using suitable procedures and animal models; for instance, as described below

### Drug treatment of mice with ischemic retinopathy

Ischemic retinopathy is produced in C57/BL6J mice by the following method: Seven-day-old mice and their mothers are placed in an airtight incubator and exposed to an atmosphere of 75 ± 3% oxygen for 5 days. Incubator temperature is maintained at 23 ± 2°C, and oxygen is measured every 8 hours with an oxygen analyzer. After 5 days, the mice are removed from the incubator, placed in room air, and COX-2 inhibitor drug treatment is begun. Drugs are diluted in phosphate-buffered saline (PBS) or 1% dimethyl sulfoxide depending upon their solubility characteristics and then diluted to their final concentration with PBS. Vehicle (control) or vehicle containing various concentrations of drug (volume = 50 µl in neonates and 100 µl in adult mice) is placed in the stomach by gavage. After 5 days of treatment at P 17, mice are sacrificed, eyes are rapidly removed and frozen in optimum cutting temperature embedding compound (OCT; Miles Diagnostics, Elkhart, IN) or fixed in 10% phosphate-buffered formalin and embedded in paraffin. Adult C57BL6J mice are also treated by gavage with drug or vehicle and after 5 days, they are sacrificed and their eyes are processed for frozen or paraffin sections.

### Quantitation of retinal neovascularization

Frozen sections (10 µm) of eyes from drug-treated and control mice are histochemically stained with biotinylated griffonia simplicifolia lectin B4 (Vector Laboratories, Burlingame, CA) which selectively binds to endothelial cells. Slides are incubated in methanol/H₂O₂ for 30 minutes at 4°C, washed with 0.05 M TBS, and incubated for 30 minutes in 10% normal porcine serum. Slides are rinsed with 0.05M Tris-buffered saline, pH 7.6 (TBS) and incubated 2 hours at room temperature with biotinylated lectin. After being rinsed with 0.05M TBS, slides are incubated with avidin coupled to peroxidase (Vector Laboratories) for 45 minutes at room temperature. After being washed for 10 minutes with 0.05 M TBS, slides are incubated with diaminobenzidine to give a brown reaction product. Some slides are counterstained with hematoxyln and all are mounted with Cytoseal.

To perform quantitative assessments, 10 µm serial sections are cut through the entire extent of each eye. The entire eye is sampled by staining sections roughly 50-60 µm apart, which provided 13 sections per eye for analysis. Lectin-stained sections are examined with an Axioskop microscope (Zeiss, Thornwood, NY) and images are digitized using a 3 CCD color video camera (IK-TU40A, Toshiba, Tokyo, Japan) and a frame grabber. Image-Pro Plus software (Media Cybernetics, Silver Spring, MD) is used to delineate lectin-stained cells on the surface of the retina and their area is measured. The mean of the 13 measurements from each eye is used as a single experimental value.

### Drug treatment of mice during retinal vascular development

Litters of newborn C57/BL6J mice are divided into treatment and control groups which received daily subcutaneous injections of 10 mg/kg of drug or vehicle, respectively. At P7 or P10, mice are anesthetized with ether, and perfused with 1 ml of phosphate-buffered saline containing 50 mg/ml of fluorescein-labeled dextran (2x10⁶ average mw, Sigma, St. Louis, MO).. The eyes are removed and fixed for 1 hour in 10% phosphate-buffered formalin. The cornea and lens are removed and the entire retina is carefully dissected from the eyecup, radially cut from the edge of the retina to the equator in all 4 quadrants, and flat-mounted in Aquamount, with photoreceptors facing upward. Flat-mounts are examined by fluorescence microscopy and images are digitized using a 3 CCD color video camera (IK-TU40A, Toshiba, Tokyo, Japan) and a frame grabber. Image-Pro Plus software (Media Cybernetics, Silver Spring, MD) is used to measure the distance from the center of the optic nerve to the leading front of developing retinal vessels in each quadrant and the mean is used as a single experimental value.

### Drug treatment of mice with choroidal neovascularization

Choroidal neovascularization is generated by modification of a previously described technique. Briefly, 4 to 5 week old male C57BL/6J mice are anesthetized with ketamine hydrochloride (100 mg/kg body weight) and the pupils are dilated with 1% tropicamide. Three burns of krypton laser photocoagulation (100 µm spot size, 0.1 seconds duration, 150 mW) are delivered to each retina using the slit lamp delivery system of a Coherent Model 920 Photocoagulator and a hand held cover slide as a contact lens. Burns are performed in the 9, 12, and 3 o'clock positions of the posterior pole of the retina. Production of a bubble at the time of laser, which indicates rupture of Bruch's membrane, is an important factor in obtaining CNV, so only mice in which a bubble is produced for all three burns are included in the study. Ten mice are randomly assigned to treatment with vehicle alone and 10 mice received vehicle containing 120µmoles/kg/day of one of the test drugs given orally by gavage. After 14 days, the mice are killed with an overdose of pentobarbital sodium, and their eyes are rapidly removed and frozen in optimal cutting temperature embedding compound (OCT).

### Quantitation of choroidal neovascularization

Frozen serial sections (10 µm) are cut through the entire extent of each burn and histochemically stained with biotinylated griffonia simplicifolia lectin B4 (Vector Laboratories, Burlingame, CA), which selectively binds to endothelial cells. Slides are incubated in methanol/H₂O₂ for 30 minutes at 4°C, washed with 0.05 M TBS, and incubated for 30 minutes in 10% normal swine serum. Slides are rinsed with 0.05M TBS and incubated 2 hours at 37°C with biotinylated lectin. After being rinsed with 0.05M TBS, slides are incubated with Streptavidin-phosphatase (Kirkegaard and Perry Laboratories, Cabin John, MD) for 30 minutes at room temperature. After a 10 minute wash in 0.05 M Tris buffer, pH 7.6, slides are developed in Histomark Red (Kirkegaard and Perry) to give a red reaction product, and mounted with Cytoseal (Stephens Scientific, Riverdale, NJ). Some slides are counterstained with Contrast Blue (Kirkegaard and Perry).

To perform quantitative assessments, lectin-stained sections are examined with an Axioskop microscope (Zeiss, Thornwood, NY) and images are digitized using a 3 CCD color video camera (IK-TU40A, Toshiba, Tokyo, Japan) and a frame grabber. Image-Pro Plus software (Media Cybernetics, Silver Spring, MD) is used to delineate and measure the area of lectin-stained blood vessels in the subretinal space. For each lesion, area measurements are made for all sections on which some of the lesion appeared and added together to give the integrated area measurement. Values are averaged to give one experimental value per mouse. A 2-sample t-test for unequal variances is performed to compare the log mean integrated area between treatment and control mice.

Compounds of formula I inhibit ocular angiogenesis when tested in the models described above.

The following examples are intended to illustrate the invention.

### EXAMPLES

### A. Formulation Examples

### Example 1

**Table 1**

| **Ingredient** | **Amount per 200 mg tablet batch (kg)** |
|---|---|
| **Core** | |
| **Granulation** | |
| 5-methyl-2-(2'-chloro-6'-fluoroanilino)phenylacetic acid drug substance | 50** |
| Microcrystalline cellulose, NF (PH 101) | 12.85 |
| Lactose monohydrate, NF | 11.65 |
| Croscarmellose sodium, NF | 1 |
| Povidone, USP | 4 |
| Titanium dioxide, USP | 2 |
| Water, purified ***, USP | 20.375 |

| **Extra-granular Phase** | |
|---|---|
| Microcrystalline cellulose, NF (PH 102) | 13 |
| Croscarmellose sodium, NF | 3 |
| Titanium dioxide, USP | 2 |
| Magnesium stearate, NF | 0.5 |

| Coating | |
|---|---|
| Opadry white | 2.801**** |
| Opadry yellow | 2.0 **** |
| Opadry red | 04**** |
| Opadry black | 0.0504 **** |
| Water, purified ***, USP | 29.758 **** |

| | |
|---|---|
| ** The weight of drug substance is taken with reference to the dried substance (100 per cent) on the basis of the assay value (factorization). The difference in weight is adjusted by the amount of microcrystalline cellulose used. *** Removed during processing. **** Includes a 50 % excess for loss during the coating process. | |

Table 1, above, sets out the formula for a batch of approximately 250,000 immediate release film-coated tablets of 5-methyl-2-(2'-chloro-6'-fluoroanilino)-phenylacetic acid. To make the tablets, titanium dioxide is dispersed in water, followed by the addition of povidone and mixing for 20 minutes to make a povidone/titanium dioxide suspension. The drug substance, lactose, microcrystalline cellulose, and croscarmellose are mixed in a high shear mixer (e.g., a Collette Gral) for 5 minutes to form a drug mixture. The drug mixture is granulated in the high shear mixer with the povidone/titanium dioxide suspension. The suspension is pumped at a rate of 3 kg/min into the drug mixture. The resulting mixture is mixed an additional 90 seconds after all the suspension is added. The wet granulation is dried in a fluid bed dryer, using an inlet air temperature of 50 °C. The residual water target is 3.5 % (with a permissible range of 2.5 - 4.5 %). The dried granulation is passed through a screen using a mill (oscillator) and a 30 mesh screen. The previous steps are repeated to make a second granulation.

The extra-granular phase titanium dioxide is passed through a 60 mesh hand screen. The dry granulations are mixed with the extra-granular phase microcrystalline cellulose, croscarmellose sodium and titanium dioxide in a twin shell mixer for 300 revolutions to form a penultimate mixture. Magnesium stearate is passed through a 60 mesh hand screen and is mixed with the penultimate mixture in a twin shell mixer for 50 revolutions to form a tableting mixture. The tableting mixture is pressed into tablets using a tablet press and oval punches.

The coating powders (Opadry) are mixed with purified water to make a 15 % w/w coating suspension. The tablets are film coated with the coating suspension in a coating pan using 60 °C to 75 °C inlet air temperature.

Table 2 sets out the contents of a 200 mg 5-methyl-2-(2'-chloro-6'-fluoroanilino) phenylacetic acid film-coated tablet.

**Table 2**

| **Ingredient** | **Theoretical amount [mg]** | **Function** |
|---|---|---|
| **Core** | | |
| 5-methyl-2-(2'-chloro-6'-fluoroanilino)phenylacetic acid drug substance | 200 | Active substance |
| Microcrystalline cellulose (PH 101) | 51.4 | Filler |
| Lactose | 46.6 | Filler |
| Povidone | 16 | Binder |
| Titanium dioxide | 8 | Color |
| Croscarmellose sodium | 4 | Disintegrant |
| Water, purified * | Q.S. | Granulating liquid |

| **Extragranular phase** | | |
|---|---|---|
| Microcrystalline cellulose (PH 102) | 52 | Filler |
| Croscarmellose sodium | 12 | Disintegrant |
| Titanium dioxide | 8 | Color |
| Magnesium stearate | 2 | Lubricant |
| Core weight | **400** | |

| **Coating** | | |
|---|---|---|
| Opadry white (00F18296) | 7.4676 | Color |
| Opadry yellow (00F12951) | 5.3312 | Color |
| Opadry red (00F15613) | 1.0668 | Color |
| Opadry black (00F17713) | 0.1344 | Color |
| Water, purified * | Q.S. | Coating solvent |
| **Total weight** | **414** | |

| | | |
|---|---|---|
| * removed during processing | | |

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1

### Example 2

An alternative formulation is as set out in Table 3, with information about as percentage w/w, mg/dose, and kg/ 50,000 tablet batch.

**Table 3 Alternative formulation composition**

| **% w/w** | **Ingredient** | **Mg/dose** | **Kg/batch** |
|---|---|---|---|
| | Granulation | | |
| 65.04 | 5-methyl-2-(2'-chloro-6'-fluoroanilino) phenylacetic acid drug substance | 400.00 | 20.00 |
| 2.15 | Croscarmellose sodium, NF (Ac-Di-Sol) | 13.22 | 0.661 |
| 6.60 | Povidone K30, USP | 40.59 | 2.029 |
| 18.12 | Purified water, USP* | Qs | Qs |

| | Blending | | |
|---|---|---|---|
| 23.56 | Microcrystalline Cellulose, NF (Avicel PH 102) | 144.90 | 6.066 |
| 2.15 | Croscarmellose sodium, NF (Ac-Di-Sol) | 13.22 | 0.553 |
| 0.50 | Magnesium Stearate, NF (vegetable source) | 3.07 | 0.128 |

| | Film Coating | | |
|---|---|---|---|
| 84.46 | Opadry, Global White 00F18296 | 15.2028 | 0.296637 |
| 14.03 | Opadry, Global Red 00F15613 | 2.5254 | 0.049275 |
| 1.51 | Opadry, Global Black 00F17713 | 0.2718 | 0.005303 |
| | Purified Water, USP* | Qs | 1.990218 |
| | Film Coated Tablet Weight | 633.00 | |

| | | | |
|---|---|---|---|
| *Does not appear in final product. Percentage of water added used for granulation based on the dry weight of drug substance and croscarmellose sodium. | | | |

The batch is granulated as described in Example 1. The granulation is dried to residual moisture (% LOD) of 1.79%. The formulation process is the same as for the development batches as described above, except for the additional step of coating with Opadry in a coating pan. The coating powders (Opadry) are mixed with purified water to make a 15 % w/w coating suspension. The tablets are film coated with the coating suspension in a coating pan using 60°C to 75°C inlet air temperature. Based on friability data, a target force of 18 KN (16 - 20 KN range) is used to compress the remainder of the batch, resulting in acceptable friability (less than 0.5%) and the disintegration times of less than 5 mins. The ejection force is approximately 800 N throughout the compression run. This demonstrates that the blend is lubricated adequately. No picking/sticking is observed on the punch surfaces after 225 minutes. Thus, a smaller size tablet with high drug loading (65%) is achieved using a high shear granulation process, using 17 X 6.7 mm ovaloid tooling to get tablets with acceptable hardness and friability characteristics.

In addition, the tablet formulations may contain 5-methyl-2-(2'-chloro-6'-fluoroanilino)benzyl alcohol and/or 5-methyl-2-(2'-chloro-6'-fluoroanilino)benzoic acid in an amount between about 0.01 and 2% by weight, more specifically between about 0.1 and 1%.

### Example 3

Wet granulated tablet composition

| Amount per tablet | Ingredient |
|---|---|
| 25 mg | COX-2 inhibitor |
| 79.7 mg | Microcrystalline cellulose |
| 79.7 mg | Lactose monohydrate |
| 6 mg | Hydroxypropyl cellulose |
| 8 mg | Croscarmellose sodium |
| 0.6 mg | Iron oxide |
| 1 mg | Magnesium stearate |

Tablet dose strengths of between 5 and 125 mg can be accomodated by varying total weight, and the ratio of the first three ingredients. Generally it is preferable to maintain a 1:1 ratio for microcrystalline cellulose: lactose monohydrate.

### Example 4

### Directly compressed tablet composition

| Amount per tablet | Ingredient |
|---|---|
| 25 mg | COX-2 inhibitor |
| 106.9 mg | Microcrystalline cellulose |
| 106.9 mg | Lactose anhydrate |
| 7.5 mg | Croscarmellose sodium |
| 3.7 mg | Magnesium stearate |

Tablet dose strengths of between 5 and 125 mg can be accomodated by varying total tablet weight, and the ratio of the first three ingredients. Generally it is preferable to maintain a 1:1 ratio for microcrystalline cellulose:lactose monohydrate.

### Example 5

### Hard gelatine capsule composition

| Amount per capsule | Ingredient |
|---|---|
| 25 mg | COX-2 inhibitor |
| 37 mg | Microcrystalline cellulose |
| 37 mg | Lactose anhydrate |
| 1 mg | Magnesium stearate |
| 1 capsule | Hard gelatin capsule |

Capsule dose strengths of between 1 and 50 mg can be accomodated by varying total fill weight, and the ratio of the first three ingredients. Generally it is preferable to maintain a 1:1 ratio for microcrystalline cellulose:lactose monohydrate.

### Example 6

### Oral solution

| Amount per 5mL Ingredient | |
|---|---|
| 50 mg | COX-2 inhibitor |
| to 5 mL with Polyethylene oxide 400 | |

### Example 7

### Oral suspension

| Amount per 5mL dose Ingredient | |
|---|---|
| 101 mg | COX-2 inhibitor |
| 150 mg | Polyvinylpyrrolidone |

### Oral suspension

| Amount per 5mL dose Ingredient | |
|---|---|
| 2.5 mg | Poly oxyethylene sorbitan monolaurate |
| 10 mg | Benzoic acid |
| to 5 mL with sorbitol solution (70%) | |

Suspension dose strengths of between 1 and 50 mg/5 ml can be accomodated by varying the ratio of the first two ingredients.

### Example 8

### Intravenous infusion

| Amount per 200 mL dose Ingredient | |
|---|---|
| 1 mg | COX-2 inhibitor |
| 0.2 mg | Polyethylene oxide 400 |
| 1.8 mg | Sodium chloride |
| to 200 mL | Purified water |

## Claims

1. Use of a compound of formula I 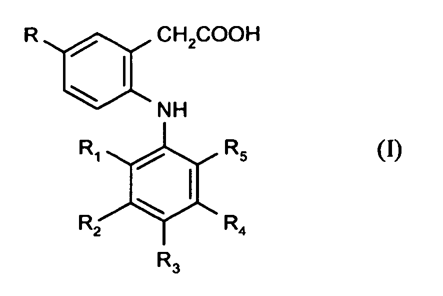 wherein
R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl;
or a pharmaceutically acceptable salt or prodrug ester thereof,
in the preparation of a medicament for use in the treatment of an angiogenesis-mediated ocular disorder, wherein said angiogenesis-mediated ocular disorder is selected from age-related macular degeneration, diabetic retinopathy and diabetic macular edema.

2. Use according to claim 1 in which the compound of : formula I is 5-methyl-2-(2-chloro-6-fluoroanilino)-phenylacetic acid or a pharmaceutically acceptable salt thereof, or a pharmaceutically acceptable prodrug ester thereof.

3. Use according to claim 1 wherein the compound of formula I is in the form of an oral composition.

4. Use according to claim 1 wherein the compound of formula I is in the form of a topical composition.

5. Use according to claim 1 wherein the angiogenesis-mediated ocular disorder is selected from age-related macular degeneration, and diabetic retinopathy.

## Patentansprüche

1. Verwendung einer Verbindung der Formel I 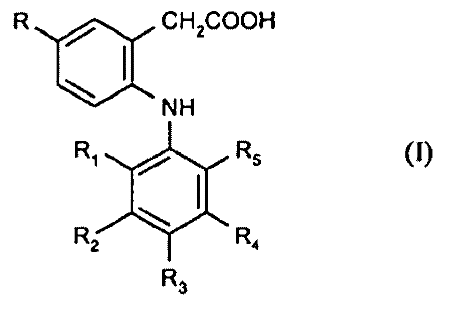 worin
R für Methyl oder Ethyl steht,
R₁ für Chlor oder Fluor steht,
R₂ für Wasserstoff oder Fluor steht,
R₃ für Wasserstoff, Fluor, Chlor, Methyl, Ethyl, Methoxy, Ethoxy oder Hydroxy steht,
R₄ für Wasserstoff oder Fluor steht, und
R₅ für Chlor, Fluor, Trifluormethyl oder Methyl steht,
oder eines pharmazeutisch akzeptablen Salzes oder Prodrugesters hiervon,
zur Herstellung eines Arzneimittels für die Behandlung einer durch eine Angiogenese mediierten Augenstörung, worin die durch eine Angiogenese mediierte Augenstörung ausgewählt ist aus einer altersbedingten Makuladegeneration, einer diabetischen Retinopathie und einem diabetischen Makulaödem.

2. Verwendung nach Anspruch 1, worin die Verbindung der Formel I 5-Methyl-2-(2-chlor-6-fluoranilino)-phenylessigsäure oder ein pharmazeutisch akzeptables Salz hiervon oder ein pharmazeutisch akzeptabler Prodrugester hiervon ist.

3. Verwendung nach Anspruch 1, worin die Verbindung der Formel I in der Form einer oralen Zusammensetzung vorliegt.

4. Verwendung nach Anspruch 1, worin die Verbindung der Formel I in der Form einer topischen Zusammensetzung vorliegt.

5. Verwendung nach Anspruch 1, worin die durch eine Angiogenese mediierte Augenstörung ausgewählt ist aus einer altersbedingten Makuladegeneration und einer diabetischen Retinopathie.

## Revendications

1. Utilisation d'un composé de formule I : 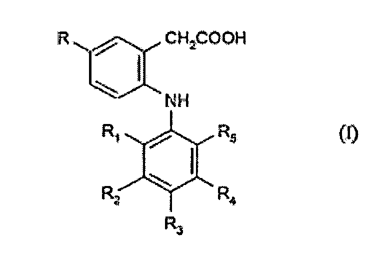 dans laquelle
R est un groupe méthyle ou éthyle ;
R₁ est un groupe chloro ou fluoro ;
R₂ représente l'hydrogène ou un groupe fluoro ;
R₃ représente l'hydrogène, un groupe fluoro, chloro, méthyle, éthyle, méthoxy, éthoxy ou hydroxy ;
R₄ représente l'hydrogène ou un groupe fluoro ; et
R₅ est un groupe chloro, fluoro, trifluorométhyle ou méthyle ;
ou de l'un de ses sels ou esters précurseurs médicamenteux pharmaceutiquement acceptables ;
pour la préparation d'un médicament à utiliser dans le traitement d'une affection oculaire liée à une angiogenèse, ladite affection oculaire liée à une angiogenèse étant choisie parmi la dégénérescence maculaire liée à l'âge, la rétinopathie diabétique et l'oedème maculaire diabétique.

2. Utilisation selon la revendication 1, dans laquelle le composé de formule I est l'acide 5-méthyl-2-(2-chloro-6-fluoroanilino)-phénylacétique ou l'un de ses sels pharmaceutiquement acceptables, ou l'un de ses esters précurseurs médicamenteux pharmaceutiquement acceptables.

3. Utilisation selon la revendication 1, dans laquelle le composé de formule I est sous forme d'une composition orale.

4. Utilisation selon la revendication 1, dans laquelle le composé de formule I est sous forme d'une composition topique.

5. Utilisation selon la revendication 1, dans laquelle l'affection oculaire liée à une angiogenèse est choisie parmi la dégénérescence maculaire liée à l'âge et la rétinopathie diabétique.
